(19) Europäisches Patentamt
European Patent Office
Office européen des brevets

(11) Veröffentlichungsnummer: **0 014 958**
**B1**

# (12) EUROPÄISCHE PATENTSCHRIFT

(45) Veröffentlichungstag der Patentschrift: **23.06.82**

(51) Int. Cl.³: **C 07 D 317/42**

(21) Anmeldenummer: **80100736.0**

(22) Anmeldetag: **14.02.80**

## (54) Verfahren zur Herstellung von 4,5-Perfluor-1,3-dioxolanen.

(30) Priorität: **20.02.79 DE 2906447**

(43) Veröffentlichungstag der Anmeldung: **03.09.80 Patentblatt 80/18**

(45) Bekanntmachung des Hinweises auf die Patenterteilung: **23.06.82 Patentblatt 82/25**

(84) Benannte Vertragsstaaten: **AT BE CH DE FR GB IT NL**

(56) Entgegenhaltungen: **DE-A-1 543 786**

(73) Patentinhaber: **HOECHST AKTIENGESELLSCHAFT, Postfach 80 03 20, D-6230 Frankfurt/Main 80 (DE)**

(72) Erfinder: **Siegemund, Günter, Dr., Frankfurter Strasse 21, D-6238 Hofheim am Taunus (DE)**
Erfinder: **Muffler, Herbert, Dr., Tucholskystrasse 14, D-6000 Frankfurt am Main 70 (DE)**

EP 0 014 958 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von 4,5-Perfluor-1,3-dioxolanen

Es ist bekannt, daß die Reaktion von Perfluoralkenepoxiden mit Ketonen zu 2,2-Dialkyl-4,5-perfluor-1,3-dioxolanen führt. Wie jedoch die Beispiele in H. S. Eleuterio, J. Macromol Sci.-Chem. A6 (6), 1027 (1972) und DE-OS 1 543 786 zeigen, ist dieses Verfahren auf Ketone als Carbonylverbindungen beschränkt. Es werden hochreaktive Epoxide, wie

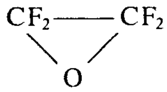

als Ausgangsstoffe benötigt, die bisher nur in verdünnter Form in Gemischen mit dem zugrunde liegenden Perfluorolefin, z. B. $CF_2=CF_2$, erzeugt werden konnten und als reine Komponente in technischem Maßstab nicht handhabbar sind. Andererseits erfordert das bekannte Verfahren bei weniger reaktionsfähigen Perfluoralkenepoxiden wie

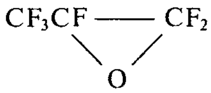

Reaktionszeiten von Tagen für Ausbeuten zwischen 16 und 39% d. Th. an 2,2-Dialkyl-4,5-perfluor-1,3-dioxolanen.

Die vorliegende Erfindung betrifft ein Verfahren zur Herstellung von 4,5-Perfluor-1,3-dioxolanen der allgemeinen Formel I

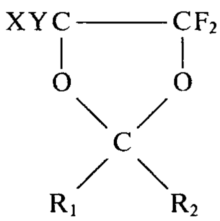

(I)

in der jeweils unabhängig voneinander X und Y Fluor oder Trifluormethyl und $R_1$ und $R_2$ Wasserstoff oder Methyl bedeuten, das dadurch gekennzeichnet ist, daß man ein 2-(α-Chloralkoxi)-perfluorcarbonsäurehalogenid der allgemeinen Formel II

$$R_1R_2CCl-O-CXY-COZ \qquad (II)$$

in der X, Y, $R_1$ und $R_2$ die für Formel I angegebene Bedeutung haben und Z Chlor oder Fluor bedeutet, mit einem Fluorid der I. Hauptgruppe des Periodensystems, mit Ammoniumfluorid oder mit einem Alkylammoniumfluorid, umsetzt.

Die Reaktion verläuft nach dem Schema

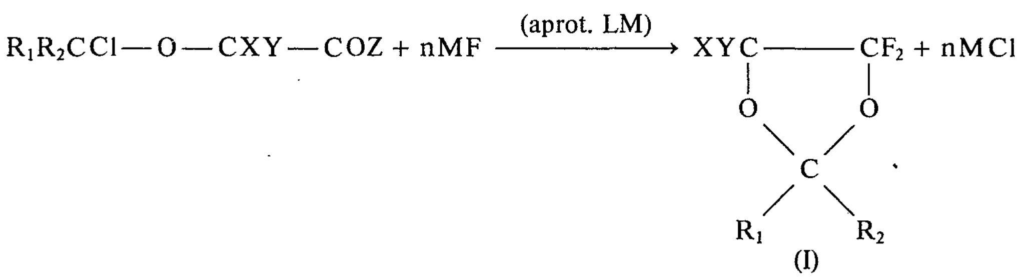


(I)

worin n = 1 ist, wenn Z für Fluor steht und n = 2 ist, wenn Z für Chlor steht. MF steht für die vorstehend genannten Fluoride.

Die für das Verfahren der vorliegenden Erfindung verwendeten Ausgangsstoffe der Formel II sind durch Lichtchlorierung von 2-Alkoxi-perfluorcarbonsäurehalogeniden zugänglich, die entweder in der Literatur beschrieben sind (vgl. DE-OS 1 793 240) oder aus den bekannten 2-Alkoxi-perfluorcarbonsäureestern durch übliche Methoden, wie Überführung in das entsprechende Natriumsalz, durch alkalische Verseifung der Ester (vgl. D. Sianesi et al., J. Org. Chem. 31, 2312—2316 [1966]) und anschließende Reaktion mit einem anorganischen Säurechlorid wie $POCl_3$ oder $SOCl_2$ hergestellt werden können.

Als Fluoride sind Alkalifluoride wie KF und CsF — in Gegenwart oder in Abwesenheit von HF oder Kronenethern — Ammoniumfluorid oder alkylsubstituierte Ammoniumfluoride wie $R'NH_2 \cdot HF$, $R_2'NH \cdot HF$, $R_3'N \cdot HF$ oder $R_4'NF$ geeignet, worin R' Methyl, Ethyl, n- oder i-Propyl oder n-Butyl bedeutet. Bevorzugt werden KF und die trialkylsubstituierten Ammoniumfluoride, $(CH_3)_3N \cdot HF$, $(C_2H_5)_3N \cdot HF$ und $(n\text{-}C_4H_9)_3N \cdot HF$ verwendet.

Das Verfahren der Erfindung kann ohne Lösungsmittel oder mit einem aprotisch-polaren Lösungsmittel, bei Temperaturen zwischen −20°C und +160°C, vorzugsweise zwischen 0°C und 120°C, insbesondere zwischen 20°C und 100°C, durchgeführt werden. Die Umsetzung erfolgt ohne Überdruck und führt in vergleichsweise kurzen Reaktionszeiten zu guten bis sehr guten Ausbeuten an 4,5-Perfluor-1,3-dioxolanen der Formel I.

Die genannten Fluoride werden in der äquivalenten Menge angewendet oder im Überschuß eingesetzt. Zweckmäßig werden sie in einem Überschuß von 10 bis 50 Mol-Prozent pro Grammatom Chlor, das pro Mol 2-(α-Chloralkoxi)-perfluorcarbonsäurehalogenid vorhanden ist, angewendet.

Gegebenenfalls können als aprotisch-polare Lösungsmittel gewählt werden: Nitrile, wie Acetonitril, n-Butyronitril oder Benzonitril, Säureamide, wie Formamid, Dimethylformamid, Hexamethylphosphorsäuretrisamid oder N-Methyl-pyrrolidon, Polyether, wie 1,4-Dioxan, Dimethoxiethan oder Diethylenglykoldimethylether (Diglyme), Dimethylsulfoxid oder Sulfolan.

Für die exotherme Reaktion ist es ohne Belang, ob das salzartige Fluorid vorgelegt, im aprotischen Lösungsmittel gelöst oder suspendiert und das 2-(α-Chloralkoxi-)-perfluorcarbonsäurehalogenid zugegeben wird oder umgekehrt das salzartige Fluorid in das 2-(α-Chloralkoxi-)-perfluorcarbonsäurehalogenid oder dessen Lösung in einem aprotischen Lösungsmittel eingetragen wird. Allerdings ist es zweckmäßig, durch gutes Rühren und äußere Kühlung die auftretende Reaktionswärme abzuführen.

Nach einer bevorzugten Arbeitsweise wird das Fluorid in einem aprotisch-polaren Lösungsmittel vorgelegt, unter Rühren und Kühlen mit einem Eisbad das 2-(α-Chloralkoxi)-perfluorcarbonsäurehalogenid zugetropft, das Reaktionsgemisch zum Rückfluß erhitzt und das gebildete 4,5-Perfluor-1,3-dioxolan abdestilliert.

Es war nun überraschend, daß die Umsetzung von 2-(α-Chloralkoxi)-perfluorcarbonsäurehalogeniden der Formel II mit Alkali- oder Ammoniumfluoriden nicht — wie zu erwarten — bei den erfindungsgemäßen Reaktionsbedingungen unter Chlor-Fluor-Austausch 2-(α-Fluoralkoxi-)perfluorcarbonsäurefluoride ($R_1R_2CF{-}O{-}CXY{-}COF$ mit $R_1$, $R_2$ = H, $CH_3$ und X, Y = F, $CF_3$) liefert, sondern die 4,5-Perfluor-1,3-dioxolane der Formel I zugänglich macht.

Die nach dem Verfahren gemäß der Erfindung hergestellten 4,5-Perfluor-1,3-dioxolane der Formel I sind farblose Flüssigkeiten mit ätherischem Geruch, die sowohl gegen saure wie auch gegen basische Hydrolyse beständig sind. In organischen Lösungsmitteln sind sie gut löslich, mit Wasser dagegen nur wenig mischbar.

Die 4,5-Perfluor-1,3-dioxolane der Formel I wirken bei Säugetieren narkotisierend oder sedierend, wenn sie inhaliert oder injiziert werden. Sie können daher als Narkotika verwendet werden. Außerdem lassen sich diese Verbindungen auch zusammen mit anderen Inhalationsanaesthetika wie Lachgas oder Diäthyläther, ferner zusammen mit anderen anaesthetischen oder therapeutischen Mitteln wie Muskelrelaxantien, Barbituraten und Plasmaexpandern einsetzen, wie es die moderne Kombinationsnarkose häufig erfordert.

## Beispiel 1

In einem 2-l-Vierhalskolben mit Thermometer, KPG-Rührer, Tropftrichter und 90 cm Vigreux-Kolonne mit Destillationsaufsatz werden 980 g (8,1 Mol) $(C_2H_5)_3N \cdot HF$ in 1250 ml trockenem Acetonitril vorgelegt. Zu dieser Lösung werden in 75 Minuten 876 g (5,4 Mol) $ClCH_2{-}O{-}CF_2{-}COF$ zugetropft, wobei die Temperatur bis auf 42°C ansteigt und $(C_2H_5)_3N \cdot HCl$ ausfällt. Dann gehen innerhalb von 5 Stunden bei langsamen Erwärmen des Reaktionsgemisches auf 86°C 610 g Destillat mit dem Siedebereich 38—80°C über. Eine anschließende fraktionierte Destillation über eine Füllkörperkolonne liefert 429 g (2,94 Mol) 4,4,5,5-Tetrafluor-1,3-dioxolan.

Siedepunkt: 41—43°C/750 mm.

Ausbeute: 54,5% d. Th.

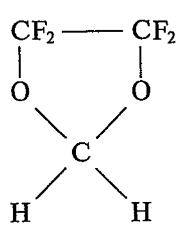

M: 146

ber.: C 24,65%; H 1,37%; F 52,0%

gef.: C 24,7%; H 1,5%; F 51,8%

Unter gleichen Reaktionsbedingungen wird ebenfalls erhalten: 4,4,5,5-Tetrafluor-2-methyl-1,3-dioxolan.
Sdp. 55°C/750 mm

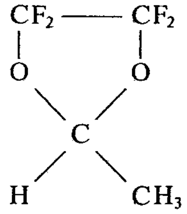

M: 160
ber.: C 30,0%; H 2,5%; F 47,5%
gef.: C 30,2%; H 2,4%; F 45,5%

Ausbeute: 85% d. Th.

Beispiel 2

In einem 500-ml-Vierhalskolben mit KPG-Rührer, Thermometer, Tropftrichter und Vigreux-Kolonne mit Destillationskopf werden 58 g (1 Mol) trockenes und pulverisiertes KF in 150 ml Acetonitril vorgelegt und 108 g (0,66 Mol) $ClCH_2-O-CF_2-COF$ zugetropft. Anschließend wird das Reaktionsgemisch auf 80° C erwärmt und das entstehende 4,4,5,5-Tetrafluor-1,3-dioxolan abdestilliert. Nach einer Reaktionszeit von 13 Stunden können im Rückstand nach Aufarbeitung mit Wasser 94% (0,62 Grammion) des zu erwartenden Chlorids nachgewiesen werden. Die Ausbeute an redestilliertem 4,4,5,5-Tetrafluor-1,3-dioxolan, Kp. 41—43° C/745 mm, beträgt 74 g (76% d. Th.).

Beispiel 3

In einem 2-l-Kolben mit KPG-Rührer, Thermometer, Tropftrichter und Vigreux-Kolonne mit Destillationsaufsatz werden zu 608 g (5 Mol) $(C_2H_5)_3N \cdot HF$, die in 700 ml trockenem Acetonitril gelöst sind, 507 g (2,21 Mol) $ClCH_2-O-CF(CF_3)COCl$ unter Rühren und Kühlen in einem Eisbad zugetropft. Das Gemisch wird 8 Stunden lang bis auf 82° C erhitzt, wobei das Produkt abdestilliert. Das gewonnene 4,5,5-Trifluor-4-trifluormethyl-1,3-dioxolan wird noch einmal rektifiziert und siedet dann bei 60° C/753 mm. Die Menge von 333 g (1,7 Mol) entspricht einer Ausbeute von 77% d. Th.

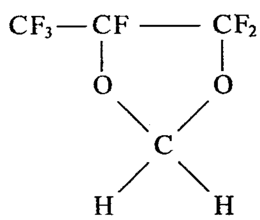

M: 196
ber.: C 24,5%; H 1,02%; F 58,1%
gef.: C 24,6%; H 1,10%; F 56,4%

Beispiel 4

In einem 1-l-Kolben mit KPG-Rührer, Thermometer, Tropftrichter und Vigreux-Kolonne mit Destillationsaufsatz wird eine Lösung von 308 g (1,5 Mol) $(n\text{-}C_4H_9)_3N \cdot HF$ in 200 ml trockenem Dimethylformamid vorgelegt. Unter Rühren werden innerhalb von 1,5 Stunden 135 g (0,59 Mol) $CF_3CF(OCH_2Cl)COCl$ zugegeben, wobei die Innentemperatur bis auf 45°C ansteigt. Es fällt kein Niederschlag an $(n\text{-}C_4H_9)_3N \cdot HCl$ aus. Beim langsamen Erwärmen auf 130° C destilliert 4,5,5-Trifluor-4-trifluormethyl-1,3-dioxolan ab. Bei vermindertem Druck (bis 10 mm Hg) kann weiteres Produkt gewonnen werden. Nach Destillation des Rohproduktes (72 g) werden 62 g oder 54% reines 4,45-Trifluor-4-trifluormethyl-1,3-dioxolan mit einem Siedepunkt von 60—61° C bei 744 mm erhalten.

Beispiel 5

In einem 1-l-Vierhalskolben mit Thermometer, Tropftrichter und Vigreuxkolonne mit Destillations- und Abnahmekopf wird eine Lösung von 182 g (1,5 Mol) $(C_2H_5)_3N \cdot HF$ in 400 ml trockenem Dioxan hergestellt. Zu dieser Lösung werden bei einer Temperatur zwischen 27 und 40°C 162 g (0,71 Mol) $CF_3CF(OCH_2Cl)COCl$ zugetropft, wobei $(C_2H_5)_3N \cdot HCl$ als Niederschlag ausfällt. Nach 5stündigem Rühren wird das 4,5,5-Trifluor-4-trifluormethyl-1,3-dioxolan abdestilliert (Fixpunkte s. Bsp. 4).
(Ausbeute: 55% d. Th.).

## Beispiel 6

In einem 2-l-Kolben mit KPG-Rührer, Thermometer, Tropftrichter und Destillationskolonne wird eine Lösung von 436 g (3,6 Mol) $(C_2H_5)_3N \cdot HF$ in 1100 ml trockenem n-Butyronitril vorgelegt. Unter Rühren und gelegentlichem Kühlen mit einem Eisbad werden 368 g (1,51 Mol) $CF_3CF(OCHCl \cdot CH_3)COCl$ bei Temperaturen zwischen 14 und 42°C zugetropft. Dabei fällt ein Niederschlag von $(C_2H_5)_3N \cdot HCl$ aus. Nach 4stündigem Rühren bei 34—38°C und Stehen über Nacht bei Raumtemperatur wird das entstandene 4,5,5-Trifluor-2-methyl-4-trifluormethyl-1,3-dioxolan abdestilliert, das noch etwas $(C_2H_5)_3N$ enthält. Nach Waschen des Destillates mit Wasser, verdünnter $H_2SO_4$ und $NaHCO_3$-Lösung sowie Trocknen über $MgSO_4$ werden bei der fraktionierten Destillation 270 g (1,28 Mol) Produkt mit dem Siedepunkt von 71—72°C mm erhalten.
Ausbeute: 85% d. Th.

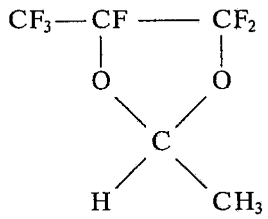

M: 210
ber.: C 28,57%; H 1,90%; F 54,28%
gef.: C 28,8%; H 1,90%; F 53,7%

Unter gleichen Reaktionsbedingungen werden erhalten:

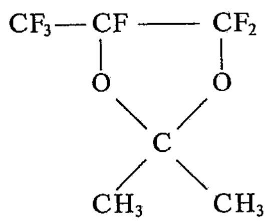

M: 224
ber.: C 32,14%; H 2,68%; F 50,89%
gef.: C 32,20%; H 2,7%; F 50,6%

Sdp. 85,4°C/761 mm
Ausbeute: 64%

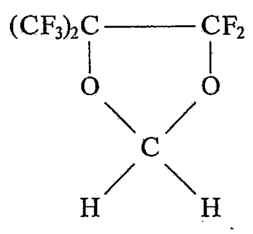

M: 246
ber.: C 24,39%; H 0,81%; F 61,79%

## Beispiel 7

Zu 206 g (1,7 Mol) $(C_2H_5)_3N \cdot HF$, die durch Erwärmen auf 55°C flüssig sind, werden innerhalb von 75 Minuten 200 g (1,13 Mol) $CH_3CHCl{-}O{-}CF_2COF$ zugetropft, wobei sich das Reaktionsgemisch bei Luftkühlung auf 71°C erwärmt. Über eine auf dem Reaktionskolben sitzende Destillationsapparatur geht dabei 2-Methyl-4,4,5,5-tetrafluor-1,3-dioxolan bei 56°C in die Vorlage über. Weiteres Erwärmen des Gemisches bis auf 162°C für 4 Stunden führt zu insgesamt 166 g Rohdestillat. Die Redestillation über eine 30 cm Raschig-Ring-Füllkörperkolonne ergibt das reine Produkt mit Sdp. 54—55°C/745 mm in einer Ausbeute von 79% d. Th. (143 g).

**Patentansprüche**

1. Verfahren zur Herstellung von 4,5-Perfluor-1,3-dioxolanen der allgemeinen Formel I,

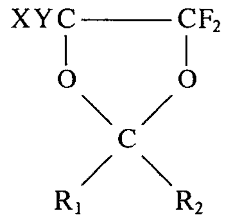

(I)

in der jeweils unabhängig voneinander X und Y Fluor oder Trifluormethyl und $R_1$ und $R_2$ Wasserstoff oder Methyl bedeuten, dadurch gekennzeichnet daß man ein 2-($\alpha$-Chloralkoxi)-perfluorcarbonsäure-halogenid der allgemeinen Formel II

$$R_1R_2CCl—O—CXY—COZ \qquad (II)$$

in der X, Y, $R_1$ und $R_2$ dieselbe Bedeutung haben wie in Formel I und Z Fluor oder Chlor bedeutet, mit einem Fluorid der I. Hauptgruppe des Periodensystems, in Gegenwart oder in Abwesenheit von HF oder Kronenethern mit Ammoniumfluorid oder mit einem Alkylammoniumfluorid, gegebenenfalls in einem aprotischpolaren Lösungsmittel, umsetzt.

2. Verfahren nach Anspruch 1, dadurch gekennzeichnet, daß als Fluorid Kaliumfluorid oder Cäsiumfluorid oder ein Alkylammoniumfluorid verwendet wird.

3. Verfahren nach Anspruch 1, dadurch gekennzeichnet, daß das Fluorid im Überschuß, vorzugsweise in einem Überschuß von 10 bis 50 Molprozent, bezogen auf die pro Mol 2-($\alpha$-Chloralkoxi)-perfluorcarbonsäurehalogenid vorhandenen Grammatome Chlor, eingesetzt wird.

4. Verfahren nach Anspruch 1, dadurch gekennzeichnet, daß als aprotisch-polare Lösungsmittel Nitrile, Säureamide, Polyether, Dimethylsulfoxid oder Sulfolan, insbesondere Acetonitril, Butyronitril, Dimethylformamid, Dioxan oder Diethylenglykoldimethylether verwendet werden.

## Claims

1. Process for the manufacture of 4,5-perfluoro-1,3-dioxolanes of the formula I

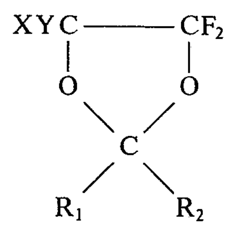

(I)

in which X and Y, independent of one another, denote fluorine or trifluoromethyl and $R_1$ and $R_2$ are hydrogen or methyl, which comprises reacting a 2-($\alpha$-chloroalkoxy)-perfluorocarboxylic acid halide of the formula II

$$R_1R_2CCl—O—CXY—COZ \qquad (II)$$

in which X, Y, $R_1$ and $R_2$ are as defined under formula I and Z denotes chlorine or fluorine, with a fluoride of an element of main group I of the Periodic Table, in the presence or absence of HF or crown ethers, with ammonium fluoride or with an alkylammonium fluoride, optionally in an aprotic-polar solvent.

2. The process of claim 1, wherein the fluoride is potassium fluoride, cesium fluoride or an alkylammonium fluoride.

3. The process of claim 1, wherein the fluoride is used in an excess of 10 to 50 mol%, calculated on the gram-atoms of chlorine present per mol of 2-($\alpha$-chloroalkoxy)-perfluorocarboxylic acid halide.

4. The process of claim 1, wherein the aprotic-polar solvent is a nitrile, an acid amide, a polyether, dimethyl sulfoxide or sulfolane, especially acetonitrile, butyronitrile, dimethyl formamide, dioxane or diethylene glycol dimethyl ether.

## Revendications

1. Procédé de préparation de 4,5-perfluoro-1,3-dioxolannes de formule générale I

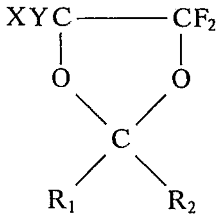

(I)

dans laquelle, chaque fois indépendamment l'un de l'autre, X et Y désignent le fluor ou un groupe trifluorométhyle et $R_1$ et $R_2$ l'hydrogène ou un groupe méthyle, caractérisé en ce qu'on fait réagir un halogénure d'acide 2-($\alpha$-chloroalcoxy)-perfluorocarboxylique de formule générale II

$$R_1R_2CCl—O—CXY—COZ \qquad (II)$$

dans laquelle X, Y, $R_1$ et $R_2$ ont la même signification que dans la formule I et Z désigne le fluor ou le chlore, avec un fluorure du premier groupe principal du tableau périodique, en présence ou en l'absence de HF ou d'étherscouronnes, avec du fluorure d'ammonium ou avec un fluorure d'alkylammonium, éventuellement dans un solvant aprotiquepolaire.

2. Procédé selon la revendication 1, caractérisé en ce qu'en tant que fluorure, on utilise du fluorure de potassium ou du fluorure de césium ou un fluorure d'alkylammonium.

3. Procédé selon la revendication 1, caractérisé en ce que le fluorure est utilisé en excès, de préférence selon un excès de 10 à 50% molaires, rapporté aux atomes grammes de chlore présents par mole d'halogénure d'acide 2-($\alpha$-chloroalcoxy)-perfluorocarboxylique.

4. Procédé selon la revendication 1, caractérisé en ce qu'on utilise en tant que solvant aprotique-polaire des nitriles, des amides d'acides, des polyéthers, du diméthylsulfoxyde ou du sulfolane, en particulier de l'acétonitrile, du butyronitrile, du diméthylformamide, du dioxanne ou l'éther diméthylique du diéthylèneglycol.